# EUROPÄISCHE PATENTSCHRIFT

(11) **EP 2 335 661 B1**
(45) Veröffentlichungstag und Bekanntmachung des Hinweises auf die Patenterteilung: **12.12.2012**
(21) Anmeldenummer: 09015673.8
(22) Anmeldetag: 18.12.2009
(51) Int. Cl.: A61F 13/02

(54) **Zuschneidehilfe für Wundauflage zur Unterdrucktherapie**
Cutting aid for wound dressing for suppression therapy
Aide à la découpe pour pansement destiné à la thérapie par dépressurisation

(43) Veröffentlichungstag der Anmeldung: 22.06.2011
(73) Patentinhaber: Paul Hartmann AG, 89522 Heidenheim (DE)
(72) Erfinder: Croizat, Pierre, Dr., 89542 Herbrechtingen (DE); Eckstein, Axel, Dr., 89522 Heidenheim (DE); Hofstetter, Jürgen, 89522 Heidenheim (DE)

(56) Entgegenhaltungen:
- US-A1- 2004 243 073
- US-A1- 2007 032 762

## Beschreibung

### Beschreibung

Die Erfindung betrifft eine Vorrichtung zur Unterdrucktherapie von Wunden umfassend ein luftundurchlässiges Abdeckmaterial zum luftdichten Verschließen der Wunde und der Wundumgebung, und ein Mittel zur funktionellen Verbindung des Wundraums mit einer außerhalb des Abdeckmaterials befindlichen Unterdruckquelle, so dass ein Unterdruck im Wundraum herstellbar ist und Flüssigkeiten aus dem Wundraum absaugbar sind.

Unter einer Wunde wird die Trennung des Zusammenhangs von Geweben der Körperhülle bei Menschen oder Tieren verstanden. Sie kann mit einem Verlust an Substanz verbunden sein.

Vorrichtungen zur Unterdrucktherapie von Wunden sind im Stand der Technik bekannt.
So beschreibt beispielsweise die WO1993/009727 eine Vorrichtung zur Förderung der Wundheilung durch die Applikation von Unterdruck auf dem die Wunde aufweisenden und die Wunde umgebenden Hautbereich. Die Vorrichtung gemäß WO1993/009727 umfasst eine Vakuumeinrichtung zur Erzeugung des Unterdrucks, eine luftdichte Abdeckung der Wunde, welche mit der Vakuumeinrichtung in einer funktionellen Verbindung steht, sowie eine Wundauflage zur Positionierung an der Wunde innerhalb der luftdichten Abdeckung. Bei der Wundauflage handelt es sich vorzugsweise um einen offenzelligen Polymer-Schaumstoff, beispielsweise um Polyester-Schaum. Die Beschaffenheit und Größe der Wundauflage kann an die individuelle Wunde angepasst werden. Ausweislich der Beschreibung von WO1993/009727 kann durch die Anwendung der Unterdrucktherapie die Wundheilung unterschiedlicher Typen von Wunden wie beispielsweise Brandwunden, Druckwunden oder Platzwunden beschleunigt werden.
Während der Behandlung kann der Unterdruck bis zum Verbandwechsel kontinuierlich aufrechterhalten werden. Alternativ kann der Unterdruck während alternierender Zyklen appliziert werden, oder es können Zyklen unterschiedlich starken Unterdrucks zur Anwendung gebracht werden.

Geräte zur Unterdrucktherapie von Wunden sind kommerziell erhältlich, beispielsweise das V.A.C.^{®}-Gerät der Firma KCI (KCI Medizinprodukte GmbH, Deutschland). Bei kommerziell erhältlichen Geräten wird oftmals eine Wundauflage eingesetzt, welche einen offenzelligen Polymer-Schaumstoff, wie beispielsweise Polyvinylalkohol (PVA) oder Polyurethan (PU), enthält. Neben der Verwendung von offenzelligem Polymer-Schaumstoff wurden andere Materialien zur Herstellung von Wundauflagen für die Unterdrucktherapie von Wunden beschrieben. So beschreibt beispielsweise die WO2001/89431 eine Kollagen-Matrix als Wundauflage für die Unterdrucktherapie von Wunden. Die GB2415908 beschreibt die Verwendung faserförmigen Materials, welches auch bioresorbierbar sein kann, in Wundauflagen für die Unterdrucktherapie von Wunden.

Wunden unterscheiden sich hinsichtlich Form und der Größe. Aus dem Stand der Technik ist bekannt, dass Wundauflagen für die Unterdrucktherapie an die individuelle Wunde angepasst werden. So gibt beispielsweise die WO1994/20041 an, dass die Wundauflage für die Unterdrucktherapie entweder auf eine passende Form und Größe zugeschnitten werden, um innerhalb der Wunde platziert zu werden. Alternativ kann nach der WO1994/20041 die Wundauflage auch ausreichend groß sein, um die umgebende Haut zu überlappen. Mit einer die gesunde Haut überlappenden Anordnung der Wundauflage soll gemäß der WO1994/20041 ein Überwachsen des Wundgewebes während der Behandlung vermieden werden. In der Praxis werden die Wundauflagen häufig in den Wundraum eingelegt, da bei einer den Wundrand überlappenden Anordnung während der Unterdrucktherapie eine Druckbelastung auf den Wundrand und die umgebende unversehrte Haut ausgeübt wird. In beiden vorgenannten Fällen muss die Wundauflage an die Form und Größe der Wunde angepasst werden. Dies erfolgt in der Praxis üblicherweise "nach Augenmaß".

Aus dem Stand der Technik sind Mittel und Verfahren zur Bereitstellung passgenauer Wundauflagen für die Unterdrucktherapie bekannt.

So schlägt die WO2008/57600 zur individuellen Bereitstellung passgenauer Wundauflagen für die Unterdrucktherapie vor, ein bioresorbierbares Polymer und porogenes System in einem Lösungsmittel aufzulösen, und nach dem Entfernen des Lösungsmittels die Wundauflage passgenau in den Wundraum einzubringen. Dabei kann die Wundauflage entweder durch manuelles Formen an die Größe und Form der Wunde angepasst werden, oder die Wundauflage kann in die Form eines Seils gebracht werden, welches in aufgerollter Form in die Wunde eingebracht wird. Das porogene System, welches im Kontakt mit Wundexsudat zu Porenbildung führt, besteht beispielsweise aus Natriumbicarbonat und Zitronensäure.

Die WO2005/009488 schlägt speziell geformte Wundauflagen für Wunden an profilierten Stellen, beispielsweise an der Ferse, vor.

Die WO2009/097308 beschreibt eine Schneidevorrichtung für Wundauflagen, insbesondere für Wundauflagen zur Verwendung in der Unterdrucktherapie.

Weiterhin sind Wundauflagen für die Unterdrucktherapie, die eine Anpassung an individuelle Wunden erleichtern sollen, kommerziell erhältlich. So enthält das V.A.C.^{®} Simplace Dressing Kit™ der Firma KCI die Schaum-Wundauflage GranuFoam™, die ohne Schneiden an die Größe der Wunde angepasst werden kann. Die Wundauflage weist eine Spiralform auf und ist in unterschiedlicher Größe erhältlich.
Die V.A.C.^{®} GranuFoam™ Standard Dressing Kits der Firma KCI enthalten Schaum-Wundauflagen in verschiedenen Formen und Größen für unterschiedliche Wunden.
Die Wundauflage V.A.C.^{®} GranuFoam™ Thin Dressing der Firma KCI wurde speziell für flache Wunden entwickelt. Sie weist Perforierungen auf, die das Zuschneiden der Wundauflage erleichtern sollen.

Der vorliegenden Erfindung liegt die Aufgabe zugrunde, die Unterdruckbehandlung von Wunden weiter zu verbessern und die Nachteile des Standes der Technik zu überwinden. Weiterhin liegt der Erfindung die Aufgabe zugrunde, Vorrichtungen und Verfahren zur Unterdrucktherapie von Wunden bereit zu stellen, mit denen eine Behandlung möglichst wirksam und möglichst schonend durchgeführt werden kann. Insbesondere soll die Aufgabe gelöst werden, die Wundauflage möglichst genau an die Form der Wunde anzupassen. Dies ist wünschenswert, da eine zu kleine Wundauflage zu Bildung von unerwünschten Hohlräumen führen kann, während eine zu groß bemessene Wundauflage zu einer Reizung der Wundränder führen kann. Die individuelle Anpassung der Wundauflage soll möglichst einfach und schnell erfolgen, um die für das Anlegen des Verbandes benötigte Zeit zu verkürzen und die Belastung des Patienten gering zu halten. Die individuelle Anpassung der Wundauflage soll weiterhin in möglichst wenigen Arbeitsschritten erfolgen, um die Sterilität der Wundauflage zu erhalten. Des Weiteren ist es für die Praxis wünschenswert, nur eine geringe Zahl unterschiedlicher Wundauflagen bereithalten zu müssen, die zur Anwendung für unterschiedlichste Wunden geeignet sind.

Die vorliegende Erfindung löst die Aufgabe durch die Bereitstellung einer Vorrichtung zur Unterdrucktherapie von Wunden umfassend
a) ein luftundurchlässiges Abdeckmaterial zum luftdichten Verschließen der Wunde und der Wundumgebung
b) ein Mittel zur funktionellen Verbindung des Wundraums mit einer außerhalb des Abdeckmaterials befindlichen Unterdruckquelle, so dass ein Unterdruck im Wundraum herstellbar ist und Flüssigkeiten aus dem Wundraum absaugbar sind
c) eine flüssigkeitsdurchlässige Wundauflage, zum Einbringen in den Zwischenraum, welcher durch Wundoberfläche und Abdeckmaterial gebildet wird,
   **dadurch gekennzeichnet, dass** die Vorrichtung weiterhin
d) eine Zuschneidehilfe für die flüssigkeitsdurchlässige Wundauflage umfasst, wobei
   d1) die Zuschneidehilfe eine transparente Folie mit einer ersten und einer zweiten Seite umfasst,
   d2) die transparente Folie auf ihrer ersten Seite beschreibbar ist, so dass der Umriss der Wunde auf die Zuschneidehilfe gezeichnet werden kann,
   d3) die transparente Folie mindestens auf der der ersten Seite gegenüber liegenden zweiten Seite eine selbstklebende Beschichtung aufweist, so dass die Zuschneidehilfe auf die im Gebrauch von der Wunde abgewandte Seite der Wundauflage haftend aufgebracht werden kann,
      sowie durch ein Verfahren zur Bereitstellung einer zugeschnittenen flüssigkeitsdurchlässigen Wundauflage zur Unterdrucktherapie von Wunden, umfassend die Schritte
      a) Bereitstellen einer Zuschneidehilfe,
      b) Bereitstellen einer flüssigkeitsdurchlässigen Wundauflage,
      c) Nachzeichnen eines Wundumrisses auf der Zuschneidehilfe,
      d) Aufkleben der Zuschneidehilfe auf die im Gebrauch von der Wunde abgewandte Seite der flüssigkeitsdurchlässigen Wundauflage, so dass ein flächiger Verbund aus Wundauflage und Zuschneidehilfe entsteht,
      e) Zuschneiden des flächigen Verbundes aus Wundauflage und Zuschneidehilfe, wobei das Zuschneiden entlang dem in Schritt c) auf die Zuschneidehilfe gezeichneten Umriss erfolgt.

Im Verfahren zur Bereitstellung einer zugeschnittenen flüssigkeitsdurchlässigen Wundauflage zur Unterdrucktherapie von Wunden werden zunächst die Komponenten a) bis d) der vorgenannten Vorrichtung zur Unterdrucktherapie von Wunden bereitgestellt. Danach kann der Wundumriss mit einem geeigneten Stift, beispielsweise mit einem handelsüblichen abwischbaren ("non-permanent") oder nicht-abwischbaren ("permanent") Folienmarker-Stift, auf die Zuschneidehilfe gezeichnet werden. Das Nachzeichnen eines Wundumrisses auf der Zuschneidehilfe kann erfolgen, während die Zuschneidehilfe dicht, beispielsweise in einem Abstand von etwa 2 cm, über der Wunde gehalten wird. Das Nachzeichnen eines Wundumrisses auf der Zuschneidehilfe kann alternativ auch erfolgen, während die Zuschneidehilfe auf die Wunde gelegt wird. Nachdem das Nachzeichnen eines Wundumrisses auf der Zuschneidehilfe erfolgt ist, wird die Zuschneidehilfe auf die im Gebrauch von der Wunde abgewandte Seite der flüssigkeitsdurchlässigen Wundauflage geklebt, so dass ein flächiger Verbund aus Wundauflage und Zuschneidehilfe entsteht. Anschließend kann der Verbund aus Wundauflage und Zuschneidehilfe entlang des aufgezeichneten Umrisses der Wunde zugeschnitten werden, wobei zum Zuschneiden vorzugsweise eine sterile Schere verwendet wird.

Die durch das erfindungsgemäße Verfahren bereit gestellte zugeschnittene flüssigkeitsdurchlässige Wundauflage weist exakt die Form der Wunde auf und kann auf die Wunde aufgelegt werden. Eine erfindungsgemäß bereit gestellte zugeschnittene Wundauflage kann die Wirksamkeit einer Unterdruckbehandlung von Wunden verbessern, da weder Hohlräume infolge einer zu klein bemessenen Wundauflage, noch eine Belastung der Wundränder infolge einer zu groß bemessenen Wundauflage auftreten. Weiterhin fühlt sich eine gemäß dem Verfahren der vorliegenden Erfindung passgenau bereit gestellte zugeschnittene Wundauflage während Unterdruckbehandlung für den Patienten angenehmer an, da Druckstellen vermieden werden können. Das erfindungsgemäße Verfahren zur Bereitstellung einer Wundauflage zur Unterdrucktherapie von Wunden ermöglich des Weiteren eine sehr schnelle und einfache Anpassung der Wundauflage an die individuelle Form und Größe der Wunde durch den Arzt bzw. durch die behandelnde Person. Eine gegebenenfalls nötige Nachbesserung einer - wie im Stand der Technik üblich - nach Augenmaß zurechtgeschnittenen Wundauflage entfällt. Dies ist für den Arzt, welcher bei der Behandlung von Wunden häufig unter Zeitdruck arbeitet, vorteilhaft. Weiterhin ist eine rasche Wundversorgung für den Patienten schonender. Das erfindungsgemäße Verfahren zur schnellen Bereitstellung einer Wundauflage zur Unterdrucktherapie von Wunden in wenigen Arbeitsschritten vermindert das Risiko, die Komponenten des Wundverbandes oder die offene Wunde zu kontaminieren.

Gemäß einer bevorzugten Ausführungsform verbleibt die Zuschneidehilfe während der Unterdruckbehandlung auf der Wundauflage. Dies ist einerseits hinsichtlich einer raschen Durchführung der Wundbehandlung in wenigen Schritten vorteilhaft. Eine auf der Wundauflage verbleibende Zuschneidehilfe kann auch hinsichtlich einer Stabilisierung des Unterdruckverbandes vorteilhaft sein, insbesondere bei großen Wunden, beispielsweise bei Wunden mit einer Fläche von mehr als 50 cm². Eine auf der Wundauflage verbleibende Zuschneidehilfe kann zudem zu einer gleichmäßigeren Druckverteilung im Wundbereich beitragen. Dabei kann eine auf der Wundauflage verbleibende Zuschneidehilfe auch einen Schutz der Wunde vor äußeren Einwirkungen während der Unterdrucktherapie bieten.

Gemäß einer weiteren bevorzugten Ausführungsform wird die Zuschneidehilfe vor der Applikation der zugeschnittenen flüssigkeitsdurchlässigen Wundauflage in die Wunde von der Wundauflage abgelöst. Ein Ablösen der Zuschneidehilfe von der zugeschnittenen Wundauflage nach erfolgter individueller Anpassung der Wundauflage kann vorteilhaft sein, falls zusätzliche Schichten innerhalb des Wundraumes unerwünscht sind. Ein Ablösen der Zuschneidehilfe von der zugeschnittenen Wundauflage kann insbesondere dann wünschenswert sein, wenn eine möglichst weiche Wundabdeckung angestrebt wird. Dies kann gegebenenfalls bei kleineren Wunden der Fall sein, beispielsweise bei Wunden mit einer Fläche von weniger als 50 cm².

Die erfindungsgemäße Vorrichtung umfasst ein Abdeckmaterial zum luftdichten Verschließen der Wunde. Unter "luftdichten Verschließen" soll hier nicht verstanden werden, dass keinerlei Gasaustausch zwischen dem Wundraum und seiner Umgebung stattfindet. Vielmehr ist mit "luftdichten Verschließen" in diesem Zusammenhang gemeint, dass unter Berücksichtigung der eingesetzten Unterdruckpumpe der für die Unterdrucktherapie von Wunden nötige Unterdruck aufrechterhalten werden kann. Es können deshalb auch Abdeckmaterialien eingesetzt werden, welche eine geringfügige Gas-Permeabilität aufweisen, so lange der für die Unterdrucktherapie notwendige Unterdruck aufrechterhalten werden kann.

Das luftdichte Abdeckmaterial kann beispielsweise in Form einer aus einem festen Material bestehenden Schale oder in Form einer flexiblen Folie vorliegen. Vorstellbar sind auch Kombinationen von festen Rahmen oder Auflageplatten mit flexiblen Folien.

In einer bevorzugten Ausführungsform der Erfindung umfasst das Abdeckmaterial zum luftdichten Verschließen der Wunde ein wasserunlösliches Polymer, oder eine Metallfolie.

In einer besonders bevorzugten Ausführungsform der Erfindung handelt es sich bei dem wasserunlöslichen Polymer um Polyurethan, Polyester, Polypropylen, Polyethylen, Polyamid oder Polyvinylchlorid, Polyorganosiloxan (Silikon), oder um eine Mischung daraus.

Dem Fachmann sind weitere geeignete polymere Folienmaterialien bekannt.

Als Abdeckmaterial können auch Fertigprodukte eingesetzt werden, welche die vorstehend genannten Eigenschaften aufweisen.

Als besonders geeignetes Abdeckmaterial für die erfindungsgemäße Vorrichtung hat sich Polyurethanfilm der Marke Hydrofilm^{®} (Paul Hartmann AG, Deutschland) oder Visulin^{®} (Paul Hartmann AG, Deutschland) erwiesen.

Das Abdeckmaterial wird in der Wundumgebung oder am Wundrand so befestigt, dass ein luftdichter Wundverschluss gewährleistet ist. Dabei kann es zweckmäßig sein, wenn das Abdeckmaterial vollflächig selbstklebend ausgestattet ist oder einen selbstklebenden Rand aufweist. Alternativ können Befestigung und Abdichtung beispielsweise mit einer Klebefolie, mit einem flüssigen Kleber oder mit einer Abdichtmasse erfolgen.

In einer bevorzugten Ausführungsform der Erfindung umfasst das Abdeckmaterial eine Folie aus einem oder mehreren wasserunlöslichen Polymeren, wobei die Folie vollflächig selbstklebend ausgestattet ist oder einen selbstklebenden Rand aufweist.

Möglich ist jedoch auch, dass das Abdeckmaterial lediglich durch den während der Unterdruckbehandlung erzeugten Unterdruck an der Wunde gehalten wird.

In einer bevorzugten Ausführungsform werden Abdeckmaterial und das Mittel zur funktionellen Verbindung des Wundraums mit einer außerhalb des Abdeckmaterials befindlichen Unterdruckquelle bereits gebrauchsfertig miteinander verbunden zur Verfügung gestellt. Es ist ganz besonders bevorzugt, dass diese Ausführungsform eine Folie aus einem oder mehreren wasserunlöslichen Polymeren enthält, welche einen selbstklebenden Rand aufweist, da diese Anordnung das Anlegen des Verbandes wesentlich erleichtert.

Die erfindungsgemäße Vorrichtung zur Unterdrucktherapie umfasst ein Mittel zur funktionellen Verbindung des Wundraums mit einer außerhalb des Abdeckmaterials befindlichen Unterdruckquelle, so dass ein Unterdruck im Wundraum herstellbar ist und Flüssigkeiten aus dem Wundraum absaugbar sind.

Der Ausdruck "Unterdruck im Wundraum" bezeichnet im Zusammenhang mit der Erfindung einen innerhalb des Wundverbandes gegenüber dem Umgebungsluftdruck (atmosphärischer Luftdruck) erniedrigten Luftdruck. Unter "innerhalb des Wundverbandes" wird der durch das Abdeckmaterial und der Wunde gebildete Zwischenraum verstanden. "Unterdruck" wird häufig auch als "negativer Druck" bezeichnet.

Die Druckdifferenz zwischen dem Luftdruck innerhalb des Wundverbandes und dem Umgebungsluftdruck wird im Zusammenhang mit der Erfindung in mm Hg (Millimeter-Quecksilbersäule) angegeben, da dies im Bereich der Unterdrucktherapie üblich ist. 1 mm Hg entspricht einem Torr beziehungsweise 133,322 Pa (Pascal). Im Zusammenhang mit der Erfindung wird der Unterdruck, d.h. die Druckdifferenz zwischen dem Luftdruck innerhalb des Wundverbandes und dem Umgebungsluftdruck, als positiver Zahlenwert in mm Hg angegeben.

In einer Ausführungsform der Erfindung handelt es sich bei dem Unterdruck um einen Unterdruck von höchstens 500 mm Hg. Dieser Unterdruckbereich von höchstens 500 mmHg hat sich als für die Wundheilung geeignet erwiesen. In einer bevorzugten Ausführungsform der Erfindung handelt es sich bei dem Unterdruck um einen Unterdruck von mindestens 10 mm Hg und höchstens 150 mm Hg.

In zwei weiteren alternativen, jeweils bevorzugten Ausführungsformen der Erfindung handelt es sich bei dem Unterdruck a) um einen konstanten Unterdruck oder b) um einen zeitlich variierenden Unterdruck.

Unter "konstantem Unterdruck" (a) wird hier verstanden, dass der Unterdruck während der Behandlung im Wesentlichen konstant gehalten wird. "Im Wesentlichen konstant" bedeutet, dass während der Behandlung geringfügige Veränderungen des Unterdrucks, beispielsweise um 15 % nach oben oder unten, auftreten können.

Ein bevorzugter konstanter Unterdruck ist der Bereich von mindestens 80 mm Hg bis höchstens 150 mm Hg.

Unter "zeitlich variierendem Unterdruck" (b) wird hier verstanden, dass der Unterdruck während der Behandlung gezielt variiert wird. Unter der gezielten Variation des Luftdrucks werden diejenigen Variationen des Luftdrucks verstanden, die einsetzen, wenn nach Anlegen des Unterdruckverbandes ein erster Sollwert für den Unterdruck erreicht wurde. Dagegen ist die erste Anstiegsphase des Unterdrucks, die nach dem Anlegen des Verbandes bis zum Erreichen des ersten Sollwertes auftritt, nicht von dem Begriff "zeitlich variierender Unterdruck" umfasst. Dies gilt analog für die am Ende der Behandlung nötige Absenkung des Luftdrucks auf den Umgebungsluftdruck, die gleichfalls nicht von dem Begriff "zeitlich variierender Unterdruck" umfasst ist.

Der "zeitlich variierende Unterdruck" ist durch den Umgebungsluftdruck nach unten und durch einen maximalen Unterdruck von 500 mm Hg, vorzugsweise 150 mm Hg, insbesondere 125 mm Hg, nach oben begrenzt. Der während der Behandlung applizierte tatsächliche Unterdruck bewegt sich innerhalb dieser durch ihre Eckwerte definierten Spanne. Der "zeitlich variierende Unterdruck" umfasst also beispielsweise einen ein- oder mehrmaligen Wechsel zwischen einem oder mehreren höheren Unterdruckwerten und einem oder mehreren geringeren Unterdruckwerten. Ebenso umfasst der "zeitlich variierende Unterdruck" einen während der Behandlung erfolgenden gezielten ein- oder mehrmaligen Wechsel zwischen dem Umgebungsdruck und einem oder mehreren höheren Unterdruckwerten.

Bei einer bevorzugten Ausführungsform beträgt der maximale Unterdruck für einen zeitlich variierenden Unterdruck 125 mm Hg. Die untere Grenze für die Variation des Unterdrucks stellt bei dieser Ausführungsform der Umgebungsluftdruck dar. Während der Behandlung variiert der Unterdruck zwischen oder innerhalb dieser Eckwerte.

Bei einer weiteren bevorzugten Ausführungsform beträgt der maximale Unterdruck für einen zeitlich variierenden Unterdruck 125 mm Hg. Die untere Grenze für die Variation des Unterdrucks beträgt bei dieser Ausführungsform 20 mm Hg. Während der Behandlung variiert der Unterdruck zwischen oder innerhalb dieser Eckwerte.

Bei beiden vorgenannten Ausführungsformen kann der Wechsel zwischen dem oberen und dem unterem Druckwert periodisch oder nicht-periodisch erfolgen. Ein periodischer Wechsel ist bevorzugt. Die Zeitintervalle, in denen der höhere Unterdruck und in denen der geringere Unterdruck oder Umgebungsdruck gehalten wird können jeweils unterschiedlich lang sein. Vorzugsweise wird ein geringerer Unterdruck länger gehalten als ein höherer Unterdruck. Geeignete Zeitintervalle, in denen jeweils eine bestimmte Unterdruckeinstellung oder der Umgebungsdruck gehalten wird sind beispielsweise 1 min, 2 min, 5 min, 10 min, 30 min, 1 h, 12h oder 24h.

Besonders bevorzugt ist ein variierender Unterdruck mit den nachstehend genannten Parametern, wobei während der Behandlung kontinuierlich in den angegebenen Zeitabständen zwischen den beiden Unterdruckwerten gewechselt wird:
Ein Unterdruck von 125 mm Hg während 2 min,
   danach
   ein Unterdruck von 20 mm Hg während 5 min.

Die erfindungsgemäße Vorrichtung zur Unterdrucktherapie von Wunden umfasst weiterhin ein Mittel zur funktionellen Verbindung des Wundraums mit einer außerhalb des Abdeckmaterials befindlichen Unterdruckquelle.

Die funktionelle Verbindung kann beispielsweise mit einer Verbindungsleitung oder mit einem Unterdruck-Anschlussstück hergestellt werden. Unterdruck-Anschlussstücke sind dem Fachmann auch unter der Bezeichnung "Port" bekannt.

Bei einer Ausführungsform handelt es sich bei dem Mittel zur funktionellen Verbindung des Wundraums mit einer außerhalb des Abdeckmaterials befindlichen Unterdruckquelle um mindestens eine Verbindungsleitung. Die mindestens eine Verbindungsleitung kann durch das Abdeckmaterial hindurchgeführt werden.

Alternativ kann die mindestens eine Verbindungsleitung unter dem Rand des Abdeckmaterials durchgeführt werden.

In beiden Fällen ist die Durchtrittsstelle luftdicht abzudichten, so dass der gewünschte Unterdruck im Verband aufrechterhalten werden kann. Als Abdichtmittel eignet sich beispielsweise eine Klebefolie, eine Klebemasse, oder ein Klebestreifen.

Bei der Verbindungsleitung kann es sich beispielsweise um einen Schlauch, um ein Rohr oder um einen anderen Körper mit einem Hohlraum handeln. Ein geeigneter Schlauch ist beispielsweise ein Silicon-Drainageschlauch.

Die Verbindungsleitung verfügt zweckmäßigerweise an dem Ende, welches sich außerhalb des Wundverbandes befindet, über einen Unterdruckadapter, um mit den weiteren Komponenten des Unterdrucksystems verbindbar zu sein.

Die Verbindungsleitung verfügt an dem Ende, welches sich innerhalb des Wundverbandes befindet, über eine Öffnung.

In einer weiteren Ausführungsform handelt es sich bei dem Mittel zur funktionellen Verbindung des Wundraums mit einer außerhalb des Abdeckmaterials befindlichen Unterdruckquelle um ein Unterdruck-Anschlussstück (Port), welches auf der Innen- oder Außenseite des Abdeckmaterials befestigt werden kann, wobei das Abdeckmaterial geeignete Öffnungen aufweist. In jedem Falle ist auf eine luftdichte Abdichtung entweder der Durchtrittsöffnung (Port innen) oder der Auflagefläche (Port außen) zu achten. Die Abdichtung kann beispielsweise mit einer Klebefolie, mit einer Klebemasse, oder mit einem Klebestreifen hergestellt werden. Es ist auch denkbar, dass der Port selbst über entsprechende Befestigungseinrichtungen, wie beispielsweise Klebeflächen, verfügt. Geeignete Unterdruck-Anschlussstücke sind kommerziell erhältlich. Dabei handelt es sich typischerweise um Unterdruck-Anschlussstücke, welche auf Außenseite des Abdeckmaterials befestigt werden.

Auch das Unterdruck-Anschlussstück verfügt zweckmäßigerweise über einen Unterdruckadapter, um mit den weiteren Komponenten des Unterdrucksystems verbindbar zu sein.

Die erfindungsgemäße Vorrichtung zur Unterdrucktherapie von Wunden umfasst weiterhin eine flüssigkeitsdurchlässige Wundauflage. Als Wundauflage kommt grundsätzlich jede aus dem Stand der Technik bekannte Wundauflage in Frage, solange einerseits während der Unterdrucktherapie ein Durchtritt von Wundexsudat durch die Wundauflage hindurch gewährleistet ist und das Material anderseits keine Neigung zum Verwachsen oder Verkleben mit dem Wundgewebe aufweist. Ein Durchtritt von Wundexsudat kann entweder dadurch erfolgen, dass die Wundauflage aus einem für Flüssigkeiten durchlässigem Material gefertigt ist. Dies kann beispielsweise bei Wundauflagen aus einem Schaummaterial der Fall sein. Ein Durchtritt von Wundexsudat kann auch dadurch gewährleistet werden, dass die Wundauflage geeignete Öffnungen oder Kanäle aufweist. Weiterhin sollte die Wundauflage zuschneidbar sein. Vorzugsweise sollte die Wundauflage mit einer Schere, insbesondere mit einer sterilen Schere zuschneidbar sein.

Eine besonders geeignete Wundauflage aus einem strukturierten Gel ist in der Deutschen Patentanmeldungen Az 102008062472.1 offenbart. Dort ist ein Wundverband für die Unterdrucktherapie beschrieben, umfassend eine Trägerschicht sowie eine auf der wundzugewandten Seite der Trägerschicht aufgebrachte Wundkontaktschicht, wobei die Wundkontaktschicht quer zu ihrer flächigen Erstreckung, die Wundkontaktschicht durchdringende Öffnungen aufweist, **dadurch gekennzeichnet, dass** die wundzugewandte Seite der Wundkontaktschicht Erhebungen und Vertiefungen aufweist und die Wundkontaktschicht lediglich im Bereich der Erhebungen eine Kontaktoberfläche mit einer Hautoberfläche bildet.

Die in den Deutschen Patentanmeldungen Az. DE102008031183.9 und Az. DE102008031182.0 beschriebenen Wundauflagen können gleichfalls als flüssigkeitsdurchlässige Wundauflage für die erfindungsgemäße Vorrichtung verwendet werden. Die Wundauflageschichten aus der DE102008031183.9 und der DE 102008031182.0 sind besonders zum Einsatz in der Granulationsphase und in der Epithelisierungsphase geeignet. Sie umfassen einen hydrophilen Polyurethanschaum mit mindestens 10 Gewichtsprozent Wasseranteil.

Ebenso kann die Wundauflage eine durchlässige Non-woven-Schicht oder einen Gittertüll umfassen. Die durchlässige Non-woven-Schicht oder der Tüll besteht vorzugsweise aus einem hydrophoben Material, beispielsweise Polyester. Der Tüll kann weiterhin mit einer Salbe ausgestattet sein.

Besonders geeignete Wundauflagen sind Salbenkompressen der Marke Hydrotüll^{®} und Atrauman^{®} (Paul Hartmann AG, Deutschland).

Das Problem einer möglichen Verwachsung oder Verklebung der Wundauflage mit dem Wundgewebe wird in einer alternativen Ausführungsform der Erfindung dadurch gelöst, dass die Wundauflage entweder vollständig aus einem bioresorbierbaren Material besteht, oder aber wundseitig mit einem bioresorbierbaren Material ausgestattet ist. Geeignete bioresorbierbare Materialien sind beispielsweise aus der DE19609551 oder aus der WO02/072163 bekannt.

Vorzugsweise, insbesondere zur Verwendung der erfindungsgemäßen Vorrichtung bei der Behandlung infizierter Wunden, weist die flüssigkeitsdurchlässige Wundauflage eine antimikrobielle Beschichtung auf. Vorzugsweise handelt es sich bei der anti-mikrobiellen Beschichtung um eine Silberbeschichtung.

Eine als Wundauflageschicht geeignete Salbenkompresse mit Silberbeschichtung ist das Handelsprodukt Atrauman Ag^{®} (Paul Hartmann AG, Deutschland).

Die Wundauflageschicht kann eine antibiotisch wirksame pharmazeutische Substanz enthalten.

Es kann auch vorgesehen sein, dass die Wundauflageschicht die Wundheilung fördernde Substanzen enthält, die während der Behandlung an die Wundoberfläche abgegeben werden. Ein Beispiel für derartige Substanzen sind Wachstumsfaktoren.

In einer weiteren Ausführungsform der Erfindung umfasst die Vorrichtung zur Unterdrucktherapie von Wunden mindestens eine zusätzliche Wundauflageschicht, welche auf der zur Wunde hin zeigenden Seite der flüssigkeitsdurchlässigen ersten Wundauflage aufgebracht wird. Dabei sind auch Schichten aus unterschiedlichen Materialien umfasst. Die zusätzliche Wundauflageschicht oder Wundauflageschichten sollten weiterhin mit der ersten Wundauflage haftend verbindbar sein, so dass sie gleichzeitig zugeschnitten werden können. Durch die haftende Verbindung soll verhindert werden, dass die erste Wundauflage und die zusätzliche Wundauflageschicht gegeneinander verrutschen. Gleichfalls sollen, falls der Einsatz von mehr als einer zusätzliche Wundauflageschicht vorgesehen ist, auch die zusätzlichen Wundauflageschichten haftend miteinander verbindbar sein. Eine Kombination mehrerer Schichten ermöglicht eine optimale Anpassung an die jeweilige Wundsituation. So kann es beispielsweise vorteilhaft sein, bei einer infizierten Wunde eine Kombination aus einer silberbeschichteten Salbenkompresse mit einem Schaumverband als erster Wundauflageschicht einzusetzen.

Die erfindungsgemäße Vorrichtung zur Unterdrucktherapie von Wunden umfasst weiterhin eine Zuschneidehilfe für die flüssigkeitsdurchlässige Wundauflage. Die Zuschneidehilfe umfasst eine transparente Folie mit einer ersten und einer zweiten Seite. Die transparente Folie ist auf ihrer ersten Seite beschreibbar, beispielsweise mit einem handelsüblichen Folienmarker-Stift. Die Beschreibbarkeit der Folie ermöglicht, dass der genaue Umriss der zu behandelnden Wunde auf die Zuschneidehilfe gezeichnet werden kann. Die transparente Folie muss über eine ausreichende Transparenz verfügen, die es erlaubt, durch die Folie hindurch den Umfang der Wunde zu erkennen, wenn die Folie an die Wunde gehalten wird. Insbesondere soll es möglich sein den Umfang der Wunde zu erkennen, wenn die Folie dicht, beispielsweise in einem Abstand von etwa 2 cm, über der Wunde gehalten wird. Vorzugsweise weist die transparente Folie eine ausreichende Transparenz auf, den Umfang der Wunde auch dann zu erkennen, wenn die Folie auf die Wunde gelegt wird. Der Fachmann kann die Transparenz durch eine geeignete Auswahl von Materialien, sowie über die Dicke des Films einstellen. Es hat sich herausgestellt, dass eine Dicke der transparenten Folie von 20 µm bis 500 µm, insbesondere von 25 µm bis 100 µm, besonders gut geeignet ist. Bei der transparenten Folie handelt es sich vorzugsweise um eine Polymerfolie, insbesondere um eine Folie aus Polyurethan, Polyester, Polypropylen, Polyethylen, Polyamid, Polyvinylchlorid, Polyorganosiloxan oder eine Mischung daraus. Gemäß einer besonders bevorzugten Ausführungsform ist die transparente Folie ein Polyurethanfilm. Auf der der ersten Seite gegenüber liegenden zweiten Seite weist die Zuschneidehilfe eine selbstklebende Beschichtung auf. Die selbstklebende Beschichtung ermöglicht, dass die Zuschneidehilfe auf die im Gebrauch von der Wunde abgewandte Seite der Wundauflage haftend aufgebracht werden kann. Die selbstklebende Beschichtung erfolgt vorzugsweise mit einem Polyacrylatkleber.

Nach einer bevorzugten Ausführungsform umfasst die Zuschneidehilfe auf der im Gebrauch zur Wunde hin zeigenden Seite weiterhin mindestens eine transparente Abdeckfolie. Die Abdeckfolie schützt dabei die selbstklebende Beschichtung der transparenten Folie. Es ist bei dieser Ausführungsform wesentlich, dass auch der Verbund aus transparenter Folie und transparenter Abdeckfolie insgesamt über eine ausreichende Transparenz verfügen, die es erlaubt, durch den Verbund hindurch den Umfang der Wunde zu erkennen, wenn der Verbund an die Wunde gehalten wird und/oder auf die Wunde gelegt wird. Geeignete Materialien für die transparente Abdeckfolie umfassen Polyurethan, Polyester, Polypropylen, Polyethylen, Polyamid, Polyvinylchlorid, Polyorganosiloxan oder eine Mischung daraus. Es hat sich he,rausgestellt, dass insbesondere ein silikonisierter Polyesterfilm als transparente Abdeckfolie geeignet ist.

Vorzugsweise umfasst die Zuschneidehilfe zwei getrennte transparente Abdeckfolienteile, die derart angeordnet sind, dass sie auf einfache Weise von der transparenten Folie abgezogen werden können, beispielsweise durch die Ausbildung mindestens einer Griffleiste. Eine Griffleiste kann beispielsweise durch eine Rückfaltung eines Abdeckfolienteils ausgebildet werden. Das andere Abdeckfolienteil kann gleichfalls über eine durch Rückfaltung gebildete Griffleiste verfügen. Alternativ kann das andere Abdeckfolienteil die Griffleiste des ersten Abdeckfolienteils zumindest abschnittsweise überfangen.

Gemäß einer weiteren vorteilhaften Ausführungsform umfasst die Zuschneidehilfe auf der im Gebrauch von der Wunde weg zeigenden Seite mindestens eine weitere Folie, welche vorzugsweise einen transparenten Polyesterfilm umfasst.

Die mindestens eine weitere Folie kann als Schutzfolie ausgebildet sein. Die Schutzfolie kann nach dem Entnehmen der Zuschneidehilfe aus der vorzugsweise sterilen Verpackung entfernt werden.

Die mindestens eine weitere Folie kann insbesondere einen transparenten Polyesterfilm mit einer ersten und einer zweiten Seite umfassen, wobei die zweite Seite des transparenten Polyesterfilms auf die erste Seite der transparenten Folie aufgebracht wird. In diesem Fall kann die weitere Folie während des Nachzeichnens eines Wundumrisses auf der transparenten Folie belassen werden. Ein Nachzeichnen des Wundumrisses erfolgt bei dieser Ausführungsform auf der ersten Seite des transparenten Polyesterfilms, während die erste Seite der transparenten Folie nicht beschriftet wird. Ein mit seiner zweiten Seite auf die erste Seite der transparenten Folie aufgebrachter transparenter Polyesterfilm kann zur Stabilisierung und zum Schutz der Zuschneidehilfe beitragen und die Handhabung der Zuschneidehilfe erleichtern.

Bei der Wundbehandlung kann es erstrebenswert sein, den Verlauf der Wundheilung zu dokumentieren. Im Zusammenhang mit der Wunddokumentation ist es wünschenswert, die Größe und den Umfang der Wunde zu Beginn der Unterdruckbehandlung festzuhalten. Die Erfinder der vorliegenden Erfindung haben herausgefunden, dass die Zuschneidehilfe gemäß einer weiteren Ausführungsform nicht nur die Bereitstellung individuell zugeschnittener Wundauflagen ermöglicht, sondern zusätzlich zur einfachen und schnellen Dokumentation der Größe und des Umfangs einer Wunde eingesetzt werden kann. Gemäß dieser vorteilhaften Ausführungsform umfasst die Zuschneidehilfe eine weitere transparente Folie mit einer ersten und einer zweiten Seite, wobei ein transparenter Polyesterfilm besonders geeignet ist. Die weitere transparente Folie ist auf ihrer ersten Seite beschreibbar, so dass der Umriss der Wunde auf die weitere transparente Folie gezeichnet werden kann. Die weitere transparente Folie weist weiterhin auf der der ersten Seite gegenüber liegenden zweiten Seite eine selbstklebende Beschichtung auf, so dass die zweite Seite der weiteren transparenten Folie mit der ersten Seite der transparenten Folie haftend verbunden werden kann. Gemäß dieser Ausführungsform wird der Umriss der Wunde auf die erste Seite der weiteren transparente Folie gezeichnet. Der Verbund aus erster und weiterer transparenter Folie wird dann auf die flüssigkeitsdurchlässige Wundauflage geklebt. Nach dem Zurechtschneiden des Verbundes aus Wundauflage, transparenter Folie und weiterer transparenter Folie wird die weitere transparente Folie von der transparenten Folie abgelöst. Die weitere transparente Folie weist die Form des Wundumrisses auf, und kann beispielsweise in eine Patientenakte geklebt werden.

Vorzugsweise ist die mindestens eine weitere Folie zweiteilig ausgebildet, wobei die zwei Teile derart angeordnet sind, dass sie auf einfache Weise von der transparenten Folie abgezogen werden können, beispielsweise durch die Ausbildung mindestens einer Griffleiste. Dies kann beispielsweise dadurch erreicht werden, dass ein erstes Teil der weiteren Folie das zweite Teil der weiteren Folie überfängt.

Es hat sich als besonders vorteilhaft erwiesen, dass die Zuschneidehilfe auf ihrer ersten Seite mindestens eine weitere Folie umfasst und weiterhin auf ihrer zweiten Seite mindestens eine transparente Abdeckfolie umfasst. Es ist auch möglich, dass die Zuschneidehilfe auf ihrer im Gebrauch von der Wunde weg zeigenden Seite mindestens eine weitere Folie umfasst und auf ihrer im Gebrauch zur Wunde hin zeigenden Seite neben der selbstklebenden Beschichtung keine weitere Schicht umfasst.

Weiterhin stellt die Erfindung ein gebrauchsfertiges Set zur Unterdruck-Wundbehandlung, umfassend ein luftundurchlässiges Abdeckmaterial zum luftdichten Verschließen der Wunde und der Wündumgebung, ein Mittel zur funktionellen Verbindung des Wundraums mit einer außerhalb des Abdeckmaterials befindlichen Unterdruckquelle, so dass ein Unterdruck im Wundraum herstellbar ist und Flüssigkeiten aus dem Wundraum absaugbar sind, eine Wundauflage aus einem flüssigkeitsdurchlässigen Material zum Einbringen in den Zwischenraum, welcher durch Wundoberfläche und Abdeckmaterial gebildet wird, sowie eine Zuschneidehilfe für eine flüssigkeitsdurchlässige Wundauflage zur Unterdruckbehandlung, zur Verfügung. Die in dem Set enthaltenen Komponenten sollen dabei steril abgepackt vorliegen. Insbesondere wird die Zuschneidehilfe, die Wundauflage, das Abdeckmaterial und das Mittel zur funktionellen Verbindung des Wundraums mit einer außerhalb des Abdeckmaterials befindlichen Unterdruckquelle steril abgepackt zur Verfügung gestellt. Vorzugsweise werden alle Komponenten, bei denen dies aus medizinischer Sicht notwendig ist, steril abgepackt zur Verfügung gestellt.

Das Set kann weitere optionale Bestandteile wie beispielsweise eine oder mehrere Wundauflageschichten, Klebemittel zum Fixieren des Verbands, Abdichtmittel zur Herstellung einer luftundurchlässigen Abdichtung des Verbandes, Drucksensoren, Verbindungselemente für Drucksensoren, zusätzliche Schläuche, Verbindungsstücke für Schläuche, Desinfektionsmittel, Hautpflegemittel, pharmazeutische Zubereitungen oder eine Gebrauchsanweisung enthalten. Besonders vorteilhafte Bestandteile des Sets sind weiterhin eine sterile Schere und ein steriler Stift. Bei dem optional in dem Set enthaltenen Stift handelt es sich insbesondere um einen Folienmarker, der zum Aufzeichnen des Wundumrisses auf die Zuschneidehilfe geeignet ist.

Vorzugsweise umfasst das Set weiterhin eine gebrauchsfertige Unterdruckeinheit. Die Unterdruckeinheit kann Komponenten wie beispielsweise eine Pumpe, ein oder mehrere Flüssigkeitsbehälter, eine Steuereinheit, eine Stromversorgung, elektrische Anschlussmitteln und Schläuche enthalten. Die Unterdruckeinheit kann auch eine Vorrichtung zur funktionellen Verbindung des Unterdruckverbandes mit einer vorhandenen stationären Unterdruckquelle enthalten.

Der Vorteil des gebrauchsfertigen Sets besteht darin, dass der Unterdruckverband schnell, standardisiert und unkompliziert angelegt werden kann. Ein weiterer Vorteil besteht darin, dass alle im Wundbereich eingesetzten Bestandteile des Sets bereits sterilisiert zur Verfügung gestellt werden können.

In einer weiteren bevorzugten Ausführungsform wird eine Zuschneidehilfe zur Anwendung in der Unterdrucktherapie bei der Behandlung einer durch eine Hauttransplantation entstandenen Wunde bereitgestellt. Die Anwendung umfasst die Behandlung von durch Spalthaut-Transplantationen und von durch Vollhaut-Transplantationen entstandenen Wunden mittels Unterdrucktherapie. Vorteilhafte Wirkungen ergeben sich durch die Möglichkeit, eine Wundauflage bereitzustellen, welche in ihrem Umriss exakt dem Umriss des Transplantates entspricht. Dies ermöglicht eine sehr gleichmäßige Druckverteilung. Bei der Anwendung der Zuschneidehilfe bei der Behandlung einer durch eine Hauttransplantation entstandenen Wunde kann das Transplantat ("Skin-Graft") durch die passend zugeschnittene Wundauflage ausreichend fixiert werden. Schädliche Scherkräfte können weitgehend vermieden werden.

Ein Verfahren zur Unterdrucktherapie von Wunden, umfasst die Schritte
a) Bereitstellen einer Vorrichtung umfassend ein luftundurchlässiges Abdeckmaterial zum luftdichten Verschließen der Wunde und der Wundumgebung, ein Mittel zur funktionellen Verbindung des Wundraums mit einer außerhalb des Abdeckmaterials befindlichen Unterdruckquelle, so dass ein Unterdruck im Wundraum herstellbar ist und Flüssigkeiten aus dem Wundraum absaugbar sind, eine flüssigkeitsdurchlässige Wundauflage, zum Einbringen in den Zwischenraum, welcher durch Wundoberfläche und Abdeckmaterial gebildet wird, wobei die Vorrichtung dadurch gekennzeichnet ist, dass die Vorrichtung weiterhin eine Zuschneidehilfe für die flüssigkeitsdurchlässige Wundauflage umfasst. Die Zuschneidehilfe umfasst eine transparente Folie mit einer ersten und einer zweiten Seite. Die transparente Folie ist auf ihrer ersten Seite beschreibbar, so dass der Umriss der Wunde auf die Zuschneidehilfe gezeichnet werden kann. Des weiteren weist die transparente Folie mindestens auf der der ersten Seite gegenüber liegenden zweiten Seite eine selbstklebende Beschichtung auf, so dass die Zuschneidehilfe auf die im Gebrauch von der Wunde abgewandte Seite der Wundauflage haftend aufgebracht werden kann.
b) Auflegen der Zuschneidehilfe auf die Wunde oder alternativ halten der Zuschneidehilfe dicht, beispielsweise 2 cm, über der Wunde
c) Nachzeichnen eines Wundumrisses
d) Aufkleben der Zuschneidehilfe auf die im Gebrauch von der Wunde abgewandte Seite einer flüssigkeitsdurchlässigen Wundauflage,
e) Zuschneiden der flüssigkeitsdurchlässigen Wundauflage zusammen mit der Zuschneidehilfe, wobei beim Zuschneiden dem in Schritt c) vorgegebenem Umriss gefolgt wird
f) Auflegen der zugeschnittenen flüssigkeitsdurchlässigen Wundauflage auf die Wunde
g) Anlegen der restlichen Bestandteile des Unterdruckverbandes an der Wunde
h) Herstellung eines Unterdruckes von höchstens 500 mm Hg im Wundraum für mindestens 30 Minuten und höchstens 7 Tage.

Vorzugsweise verbleibt die Zuschneidehilfe in Schritt f) auf der im Gebrauch von der Wunde abgewandten Seite einer zugeschnittenen flüssigkeitsdurchlässige Wundauflage. Sie kann jedoch gemäß einer alternativen Ausführungsform auch vor dem Auflegen der zugeschnittenen Wundauflage von der zugeschnittenen Wundauflage abgelöst werden.

### Figuren

Nachstehend wird die erfindungsgemäße Vorrichtung zur Unterdrucktherapie von Wunden anhand von Zeichnungen näher erläutert. Die Erfindung soll jedoch nicht auf die in den Zeichnungen oder in der Beschreibung der Zeichnung dargestellten Ausgestaltungen reduziert verstanden werden. Vielmehr umfasst die erfindungsgemäße Vorrichtung auch Kombinationen der Einzelmerkmale der alternativen Formen.
**Figur 1** Schematische Darstellung eines an der Wunde angelegten Unterdruckverbandes (Seitenansicht)
**Figur 2** Schematische Darstellung einer Ausführungsform der Zuschneidehilfe (Seitenansicht).
**Figur 3** Schematische Darstellung einer weiteren Ausführungsform der Zuschneidehilfe (Seitenansicht)
**Figur 4** Schematische Darstellung einer weiteren Ausführungsform der Zuschneidehilfe (Seitenansicht)
**Figur 5** Schematische Darstellung einer bevorzugten Ausführungsform einer an eine Wunde angelegten Vorrichtung zur Unterdrucktherapie von Wunden (Seitenansicht)

### Figurenlegende

- W: Wundgrund
- 1: Wundumgebung (d.h. in der Regel unversehrte Haut)
- 2: Luftundurchlässiges Abdeckmaterial
- 3: In eine Wunde eingelegte flüssigkeitsdurchlässige Wundauflage
- 3p: Zugeschnittene flüssigkeitsdurchlässige Wundauflage
- 4: Unterdruck- Anschlussstück (Port)
- 5: Unterdruck-Verbindungsleitung
- 6: Sammelgefäß
- 7: Unterdruckeinheit
- 8: Transparente Folie
- 9: Selbstklebende Beschichtung auf transparenter Folie 8
- 10: Zuschneidehilfe
- 11, 12: Transparente Abdeckfolien
- 13, 14: Griffleisten an transparente Abdeckfolie, wobei die Griffleiste 13 durch Rückfaltung der Abdeckfolie 12 gebildet wird und Griffleiste 14 durch einen die Griffleiste 13 überfangenden Teil der Abdeckfolie 11 gebildet wird
- 15: Applikationsfolie
- 16: Selbstklebende Beschichtung auf Applikationsfolie 15

### Beschreibung der Figuren

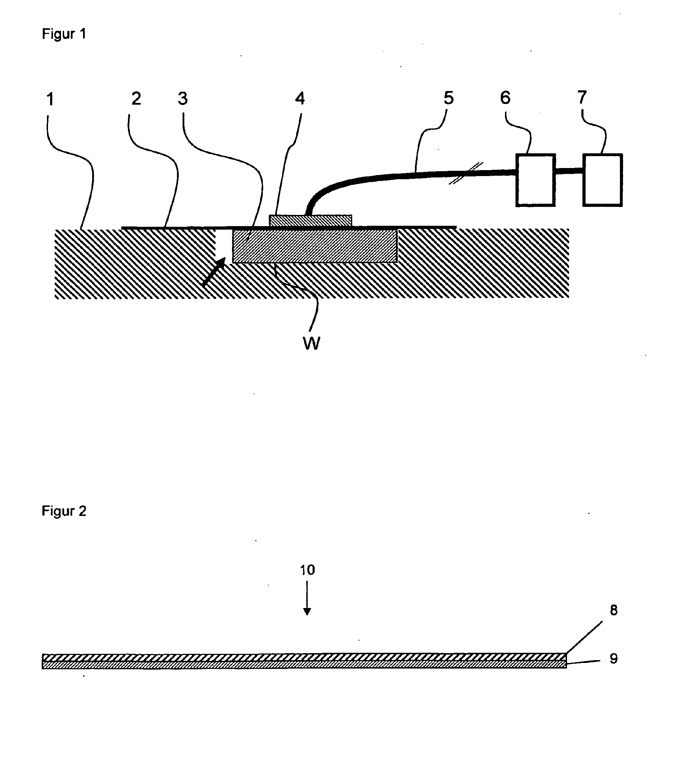

Figur 1 zeigt einen typischen Aufbau eines an der Wunde angelegten Unterdruckverbandes. Derartige Vorrichtungen sind in vielen Variationen aus dem Stand der Technik bekannt. Die Vorrichtung umfasst ein luftundurchlässiges Abdeckmaterial 2, Mittel zur funktionellen Verbindung des Wundraums mit einer außerhalb des Abdeckmaterials befindlichen Unterdruckquelle 7, einen Sammelkanister für das Wundexsudat 6, sowie eine flüssigkeitsdurchlässige Wundauflage 3, welche sich im Wundraum befindet. Üblicherweise wird die flüssigkeitsdurchlässige Wundauflage nach Augenmaß zurechtgeschnitten. Falls eine an die Größe und Form der Wunde nur unzureichend angepasste Wundauflage den Wundraum nicht vollständig ausfüllt, kann es zur Ausbildung von Hohlräumen zwischen Wundgrund W und Abdeckmaterial 2 kommen (angedeutet durch Pfeil in Figur 1). Weiterhin kann eine zu groß bemessene Wundauflage die Wundränder überlappen (in Figur 1 nicht dargestellt). Das Mittel zur funktionellen Verbindung des Wundraums mit einer außerhalb des Abdeckmaterials befindlichen Unterdruckquelle 7 umfasst in dem hier gezeigten Beispiel ein als Port bezeichnetes Unterdruck-Anschlussstück 4 und eine Unterdruckleitung 5. Der Port 4 befindet sich bei dem hier gezeigten Beispiel auf der von der Wunde weg weisenden Außenseite des luftundurchlässigen Abdeckmaterials 2. Um den Wundraum mit einer außerhalb des Abdeckmaterials befindlichen Unterdruckeinheit 7 funktionell zu verbinden, müssen sich bei dieser Anordnung im Bereich des Unterdruck-Anschlussstücks 4 eine oder mehrere durch das Abdeckmaterial 2 hindurchgehende Öffnungen befinden (nicht gezeigt). Weiterhin ist eine luftdichte Abdichtung zu gewährleisten. Eine solche Abdichtung kann beispielsweise hergestellt werden, indem auf der von der Wunde wegweisenden Oberseite des Ports eine Folie (in Figur 1 nicht gezeigt) aufgebracht wird, die mit dem Abdeckmaterial 2 verklebt wird. Das Anlegen des Verbandes kann erleichtert werden, wenn ein Port verwendet wird, bei dem ein Befestigungs- und Abdichtmittel zum Befestigen des Ports auf dem Abdeckmaterial bereits vorhanden ist. Zwischen Unterdruck-Anschlussstück 4 und Unterdruckeinheit 7 befindet sich ein Sammelgefäß 6. Das Abdeckmaterial 2 ist im Bereich der Wundumgebung 1, welche üblicherweise unversehrte Haut aufweist, befestigt. Die Größe des Abdeckmaterials sollte so bemessen sein, dass das Abdeckmaterial außerhalb des Wundbereichs im Bereich der Wundumgebung 1 befestigt werden kann. Das Abdeckmaterial 2 kann in verschiedenen Abmessungen und Formen vorliegen, beispielsweise kreisförmig, oval oder rechteckig. Es kann auch in einer an die Wunde angepassten unregelmäßigen Form vorliegen. Bei dem Abdeckmaterial 2 kann es sich um ein undurchsichtiges Material, um ein teilweise durchsichtiges Material oder um ein vollständig durchsichtiges Material handeln. Die Verwendung durchsichtigen Materials kann vorteilhaft sein, um eine Kontrolle des Heilungsverlaufs der Wunde zu ermöglichen. Die Verwendung von nur teilweise durchsichtigem oder undurchsichtigem Material kann vorteilhaft sein, um dem Patienten den Anblick der Wunde zu ersparen. Alternativ kann es sich bei dem Abdeckmaterial 2 auch um ein starres Material, welches in Form einer zur Wunde hin geöffneten Schale über den Wundbereich angebracht und im Bereich der Wundumgebung 1 befestigt wird. Das Abdeckmaterial 2 muss im Bereich der Wundumgebung 1 befestigt und luftdicht abgedichtet werden. Dies kann beispielsweise dadurch erfolgen, dass das Abdeckmaterial 2 einen Kleberand aufweist. Der Kleberand sollte möglichst bis zum Anlegen des Verbandes durch Schutzstreifen geschützt sein. Alternativ kann eine klebende Substanz entweder auf den Rand des Abdeckmaterials 2 und/oder auf die intakte Haut im Bereich der Wundumgebung aufgebracht werden. Dies hat den Vorteil, dass eine Anpassung des Abdeckmaterials an die Form und Größe der Wunde leichter möglich ist. Eine Befestigung und luftdichte Abdichtung der Vorrichtung kann aber auch durch die Verwendung von Klebestreifen oder einer Klebemasse erreicht werden. Das Anlegen des Verbandes kann durch die Verwendung eines Ports erleichtert werden, bei dem das Befestigungs- und Abdichtmittel zum Befestigen des Ports auf dem Abdeckmaterial bereits vorhanden ist. Dies ist beispielsweise bei dem im Handel erhältlichen PPM-Drainageport der Firma Phametra-Pharma und Medica-Trading GmbH (Deutschland) der Fall.

Figur 2 zeigt eine Ausführungsform der Zuschneidehilfe 10 in der Seitenansicht. Die Zuschneidehilfe 10 umfasst eine transparente Folie 8, welche auf ihrer ersten Seite beschreibbar ist, so dass der Umriss einer Wunde darauf gezeichnet werden kann. Nachdem der Umriss einer Wunde auf die erste Seite der transparenten Folie 8 gezeichnet wurde, wird die Zuschneidehilfe 10 auf die flüssigkeitsdurchlässige Wundauflage geklebt. Hierzu weist die transparente Folie 8 auf ihrer zweiten Seite eine selbstklebende Beschichtung 9 auf. Die selbstklebende Beschichtung 9 ermöglicht auf einfache Weise ein Aufkleben der Zuschneidehilfe 10 auf die Wundauflage 3. Anschließend kann die flüssigkeitsdurchlässige Wundauflage 3 zusammen mit der Zuschneidehilfe 10 zugeschnitten werden, wobei beim Zuschneiden dem auf der ersten Seite der transparenten Folie 8 gezeichneten Umriss gefolgt wird.

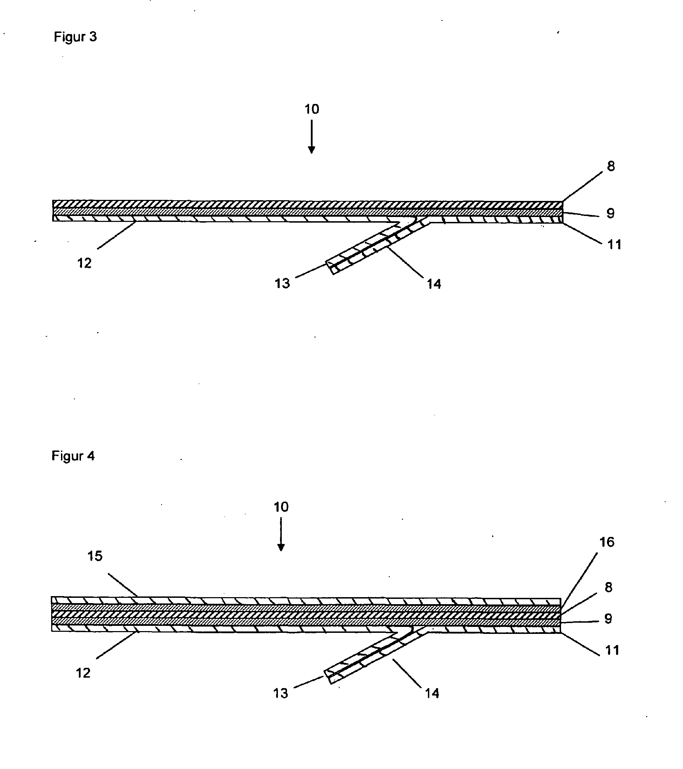

Der Aufbau einer weiteren bevorzugten Ausführungsform der Zuschneidehilfe 10 ist in Figur 3 dargestellt. Die Vorrichtung unterscheidet sich von der in Fig. 2 gezeigten Vorrichtung dadurch, dass die Klebeschicht 9, welche sich auf der zweiten Seite der transparenten Folie 8 befindet, durch die transparenten Abdeckfolien 11 und 12 bedeckt ist. Alternativ kann die Zuschneidehilfe 10 auch lediglich eine transparente Abdeckfolie, oder aber mehr als zwei transparente Abdeckfolien umfassen (nicht dargestellt). Die in Figur 3 gezeigten transparenten Abdeckfolien 11 und 12 umfassen die Griffleisten 13 und 14, die durch Rückfaltung (Griffleiste 13), bzw. Überfangen der anderen transparenten Folie (Griffleiste 14) gebildet werden. Die Griffleisten 13 und 14 erleichtern die Ablösung der transparenten Abdeckfolien 11 und 12. Dem Fachmann sind weitere Mittel bekannt, mittels denen die Ablösung der mindestens einen transparenten Abdeckfolie erleichtert werden kann.
Eine Zuschneidehilfe, welche mindestens eine transparente Abdeckfolie umfasst, ist zur Verwendung in den erfindungsgemäßen Verfahren besonders geeignet. Einerseits erleichtert die mindestens eine transparente Abdeckfolie die Handhabung der Zuschneidehilfe 10, da die Klebefläche 9 abgedeckt ist. Weiterhin stabilisiert die mindestens eine transparente Abdeckfolie die Zuschneidehilfe 10, so dass sie weniger leicht abknickt. Schließlich schützt die mindestens eine transparente Abdeckfolie die Klebefläche 9 der transparenten Folie 8 vor Wundexsudat. Eine Berührung mit Wundexsudat kann beispielsweise versehentlich erfolgen, wenn die Zuschneidehilfe dicht an die Wunde gehalten wird. Eine Berührung mit Wundexsudat erfolgt notwendigerweise, wenn die Zuschneidehilfe 10 zur Aufzeichnung des Wundumrisses auf die Wunde gelegt wird. Es ist daher vorteilhaft, die mindestens eine transparente Abdeckfolie erst dann von der Zuschneidehilfe zu lösen, wenn die Zuschneidehilfe auf die Wundauflage 3 geklebt wird.

Eine besonders vorteilhafte Ausführungsform der Zuschneidehilfe 10 wird in Figur 4 gezeigt. Bei dieser Ausführungsform umfasst die transparente Folie 8 weiterhin auf ihrer ersten Seite einen transparenten Polyesterfilme 15, welcher mit einer selbstklebenden Beschichtung 16 versehen ist. Der transparente Polyesterfilm 15 kann auch mehrteilig ausgebildet sein (in Figur 4 nicht dargestellt). Weiterhin können am transparenten Polyesterfilme 15 eine oder mehrere Griffleisten angebracht sein, um das Abziehen des Polyesterfilms 15 von der transparenten Folie 8 zu erleichtern (in Figur 4 nicht dargestellt). Das Abziehen des Polyesterfilms 15 könnte weiterhin durch eine Rückfaltung erleichtert werden (in Figur 4 nicht dargestellt). Dem Fachmann sind weitere Mittel bekannt, mittels derer die Ablösung des mindestens einen transparenten Polyesterfilms erleichtert werden kann.

Ein Vorteil der in Figur 4 gezeigten Ausführungsform besteht darin, dass der transparente Polyesterfilm bei der Anwendung zunächst auf der transparenten Folie 8 belassen werden kann. Der Wundumriss kann dabei direkt auf den transparenten Polyesterfilm 15 gezeichnet werden. Der transparente Polyesterfilm 15 kann nach dem Zuschneiden der Wundauflage von der transparenten Folie 8 abgezogen werden und zur Dokumentation der Wundgröße als Dokumentationshilfe archiviert werden kann.

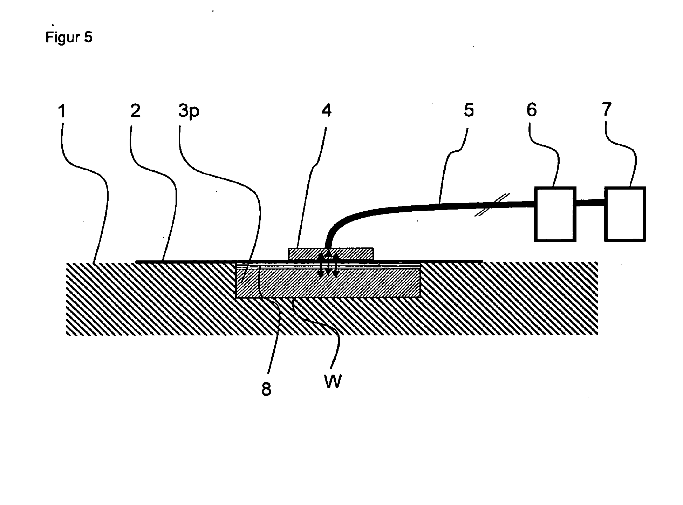

Figur 5 zeigt in bevorzugter Ausführungsform eine an eine Wunde angelegte Vorrichtung zur Unterdrucktherapie von Wunden mit bereits zugeschnittener flüssigkeitsdurchlässiger Wundauflage 3p. Die Vorrichtung umfasst ein luftundurchlässiges Abdeckmaterial 2, die Mittel 4 und 5 zur funktionellen Verbindung des Wundraums mit einer außerhalb des Abdeckmaterials befindlichen Unterdruckquelle 7, einen Sammelkanister für das Wundexsudat 6, sowie eine zugeschnittene flüssigkeitsdurchlässige Wundauflage 3p, welche sich im Wundraum auf dem Wundgrund W befindet. Gemäß der in Figur 5 gezeigten Ausführungsform wird die transparente Folie 8 nach dem Zuschneiden des Verbundes aus transparenter Folie 8 und Wundauflage 3 auf der zugeschnittenen Wundauflage 3p belassen. Der passgenau auf die Größe und Umriss der individuellen Wunde zugeschnittene Verbund aus transparenter Folie 8 und Wundauflage 3p wird in den Wundraum eingelegt. Der Wundraum wird mit einem luftundurchlässigen Abdeckmaterial 2 abgeschlossen. Um den Wundraum mit einer außerhalb des Abdeckmaterials befindlichen Unterdruckeinheit 7 funktionell zu verbinden, müssen sich bei dieser Anordnung im Bereich des Unterdruck-Anschlussstücks 4 eine oder mehrere durch das Abdeckmaterial 2 und die transparente Folie 8 hindurchgehende Öffnungen befinden (in Figur 5 durch doppelseitige Pfeile angedeutet). Üblicherweise werden diese Öffnungen nach dem Anlegen des luftundurchlässigen Abdeckmaterials 2 angebracht. Dazu kann mit einem scharfen Gegenstand, beispielsweise mit einem sterilen Skalpell, innerhalb des vom Unterdruck-Anschlussstück 4 abgedeckten Bereichs mindestens eine Öffnung durch das Abdeckmaterial 2 und die transparente Folie 8 geschnitten werden. Die mindestens eine Öffnung kann beispielsweise in der Form eines oder mehrerer kreuzförmiger Schlitze angebracht werden. Es ist jedoch besonders vorteilhaft, wenn die Öffnungen in der Form einer oder weniger (beispielsweise 2 bis 5) kreisförmiger Öffnungen mit jeweils ca. 3 - 8 mm Durchmesser durch das Abdeckmaterial 2 und die transparente Folie 8 geschnitten werden. Dabei sollte das herausgeschnittene Folien-Material möglichst vom Verband entfernt werden, da es ansonsten während der Unterdruck-Therapie das Unterdruck-Anschlussstück verstopfen könnte.

## Patentansprüche

1. Vorrichtung zur Unterdrucktherapie von Wunden umfassend
a) ein luftundurchlässiges Abdeckmaterial (2) zum luftdichten Verschließen der Wunde und der Wundumgebung (1)
b) ein Mittel (4, 5) zur funktionellen Verbindung des Wundraums mit einer außerhalb des Abdeckmaterials befindlichen Unterdruckquelle, so dass ein Unterdruck im Wundraum herstellbar ist und Flüssigkeiten aus dem Wundraum absaugbar sind
c) eine flüssigkeitsdurchlässige Wundauflage (3), zum Einbringen in den Zwischenraum, welcher durch Wundoberfläche (W) und Abdeckmaterial (2) gebildet wird,
**dadurch gekennzeichnet, dass** die Vorrichtung weiterhin
d) eine Zuschneidehilfe (10) für die flüssigkeitsdurchlässige Wundauflage (3) umfasst, wobei
d1) die Zuschneidehilfe (10) eine transparente Folie (8) mit einer ersten und einer zweiten Seite umfasst,
d2) transparente Folie (8) auf ihrer ersten Seite beschreibbar ist, so dass der Umriss einer Wunde auf die transparente Folie (8) gezeichnet werden kann,
d3) die transparente Folie (8) mindestens auf der der ersten Seite gegenüber liegenden zweiten Seite eine selbstklebende Beschichtung (9) aufweist, so dass die Zuschneidehilfe (10) auf die im Gebrauch von der Wunde abgewandte Seite der Wundauflage (3) haftend aufgebracht werden kann.

2. Vorrichtung zur Unterdrucktherapie von Wunden nach Anspruch 1, wobei die transparente Folie (8) auf der zweiten Seite mit einem Polyacrylatkleber beschichtet ist.

3. Vorrichtung zur Unterdrucktherapie von Wunden nach Anspruch 1 oder 2, wobei die transparente Folie (8) eine Polymerfolie ist.

4. Vorrichtung zur Unterdrucktherapie von Wunden nach Anspruch 3, wobei das Polymer der Polymerfolie Polyurethan, Polyester, Polypropylen, Polyethylen, Polyamid, Polyvinylchlorid, Polyorganosiloxan oder eine Mischung daraus umfasst.

5. Vorrichtung zur Unterdrucktherapie von Wunden nach einem der vorangehenden Ansprüche, wobei die Zuschneidehilfe (10) weiterhin auf der im Gebrauch zur Wunde hin zeigenden Seite mindestens eine transparente Abdeckfolie (11, 12) umfasst.

6. Vorrichtung zur Unterdrucktherapie von Wunden nach Anspruch 5, wobei die mindestens eine Abdeckfolie (11, 12) einen silikonisierten Polyesterfilm umfasst.

7. Vorrichtung zur Unterdrucktherapie von Wunden nach einem der vorangehenden Ansprüche, wobei die Zuschneidehilfe (10) auf der im Gebrauch von der Wunde weg zeigenden Seite mindestens eine weitere Folie umfasst.

8. Vorrichtung zur Unterdrucktherapie von Wunden nach Anspruch 7, wobei die mindestens eine weitere Folie einen transparenten Polyesterfilm (15) mit einer ersten und einer zweiten Seite umfasst, und wobei weiterhin
d2) der transparente Polyesterfilm (15) auf seiner ersten Seite beschreibbar ist, so dass der Umriss einer Wunde auf den transparenten Polyesterfilm (15) gezeichnet werden kann,
d3) der transparente Polyesterfilm (15) auf der der ersten Seite gegenüber liegenden zweiten Seite eine selbstklebende Beschichtung (16) aufweist, so dass der transparente Polyesterfilm (15) auf die erste Seite der transparenten Folie (8) haftend aufgebracht werden kann und der transparente Polyesterfilm (15) weiterhin nach dem Zuschneiden der Wundauflage (3) von der transparenten Folie (8) abgezogen werden und zur Dokumentation der Wundgröße als Dokumentationshilfe archiviert werden kann.

9. Verfahren zur Bereitstellung einer zugeschnittenen flüssigkeitsdurchlässigen Wundauflage (3p) zur Unterdrucktherapie von Wunden, umfassend die Schritte
a) Bereitstellen einer Zuschneidehilfe (10) nach einem der Ansprüche 1 bis 8
b) Bereitstellen einer flüssigkeitsdurchlässigen Wundauflage (3)
c) Nachzeichnen eines Wundumrisses auf der Zuschneidehilfe (10)
d) Aufkleben der zweiten Seite der Zuschneidehilfe auf die im Gebrauch von der Wunde abgewandte Seite der flüssigkeitsdurchlässigen Wundauflage (3), so dass ein flächiger Verbund aus Wundauflage (3) und Zuschneidehilfe (10) entsteht
e) Zuschneiden des flächigen Verbundes aus Wundauflage (3) und Zuschneidehilfe (10), wobei das Zuschneiden entlang dem in Schritt c) auf die Zuschneidehilfe (10) gezeichneten Umriss erfolgt.

10. Verfahren zur Bereitstellung einer zugeschnittenen flüssigkeitsdurchlässigen Wundauflage (3p) zur Unterdrucktherapie von Wunden nach Anspruch 9., weiterhin umfassend den Schritt f) Entfernen der Zuschneidehilfe (10) von der zugeschnittenen Wundauflage (3p).

11. Verfahren zur Bereitstellung einer zugeschnittenen flüssigkeitsdurchlässigen Wundauflage (3p) zur Unterdrucktherapie von Wunden nach Anspruch 9. oder 10., wobei das Nachzeichnen des Wundumrisses auf der Zuschneidehilfe (10) erfolgt, während die Zuschneidehilfe (10) an die Wunde gehalten oder auf die Wunde gelegt wird.

12. Vorrichtung nach Anspruch 1 bis 8 oder nach den Verfahren der Ansprüche 9 bis 11 bereit gestellte zugeschnittene flüssigkeitsdurchlässige Wundauflage (3p) zur Verwendung bei der Unterdrucktherapie von Wunden.

13. Gebrauchsfertiges Set zur Unterdruck-Wundbehandlung umfassend,
a) ein luftundurchlässiges Abdeckmaterial (2) zum luftdichten Verschließen der Wunde und der Wundumgebung
b) ein Mittel (4, 5) zur funktionellen Verbindung des Wundraums mit einer außerhalb des Abdeckmaterials (2) befindlichen Unterdruckquelle (7), so dass ein Unterdruck im Wundraum herstellbar ist und Flüssigkeiten aus dem Wundraum absaugbar sind
c) eine flüssigkeitsdurchlässige Wundauflage (3), zum Einbringen in den Zwischenraum, welcher durch Wundoberfläche und Abdeckmaterial (2) gebildet wird
d) eine Zuschneidehilfe (10) umfassend eine transparente Folie (8) mit einer ersten und einer zweiten Seite, welche auf ihrer ersten Seite beschreibbar ist und welche auf ihrer zweiten Seite eine selbstklebende Beschichtung (9) aufweist,
wobei die Komponenten a) bis d) steril abgepackt vorliegen.

## Claims

1. Device for vacuum therapy of wounds, comprising
a) an air-impermeable cover material (2) for air-tight closure of the wound and of the wound environment (1),
b) a means (4, 5) for functionally connecting the wound space to a vacuum source located outside the cover material, such that a vacuum can be produced in the wound space and liquids can be suctioned from the wound space,
c) a liquid-permeable wound dressing (3) for insertion into the gap formed between wound surface (W) and cover material (2), **characterized in that** the device further comprises
d) a cutting-to-size aid (10) for the liquid-permeable wound dressing (3), wherein
d1) the cutting-to-size aid (10) comprises a transparent film (8) with a first and a second side,
d2) the transparent film (8) is inscribable on its first side, such that the outline of a wound can be drawn onto the transparent film (8),
d3) the transparent film (8) has, at least on the second side opposite the first side, a self-adhesive coating (9), such that the cutting-to-size aid (10) can be adhesively mounted on that side of the wound dressing (3) facing away from the wound during use.

2. Device for vacuum therapy of wounds according to Claim 1, wherein the transparent film (8) is coated on the second side with a polyacrylate adhesive.

3. Device for vacuum therapy of wounds according to Claim 1 or 2, wherein the transparent film (8) is a polymer film.

4. Device for vacuum therapy of wounds according to Claim 3, wherein the polymer of the polymer film comprises polyurethane, polyester, polypropylene, polyethylene, polyamide, polyvinyl chloride, polyorganosiloxane or a mixture of these.

5. Device for vacuum therapy of wounds according to one of the preceding claims, wherein the cutting-to-size aid (10) further comprises at least one transparent cover film (11, 12) on the side facing towards the wound during use.

6. Device for vacuum therapy of wounds according to Claim 5, wherein the at least one cover film (11, 12) comprises a siliconized polyester film.

7. Device for vacuum therapy of wounds according to one of the preceding claims, wherein the cutting-to-size aid (10) comprises at least one further film on the side facing away from the wound during use.

8. Device for vacuum therapy of wounds according to Claim 7, wherein the at least one further film comprises a transparent polyester film (15) having a first and a second side, and wherein furthermore d2) the transparent polyester film (15) is inscribable on its first side, such that the outline of a wound can be drawn onto the transparent polyester film (15),
d3) the transparent polyester film (15) has, on the second side opposite the first side, a self-adhesive coating (16), such that the transparent polyester film (15) can be mounted adhesively on the first side of the transparent film (8) and, after the wound dressing (3) has been cut to size, the transparent polyester film (15) can furthermore be withdrawn from the transparent film (8) and archived as a documentation aid for documenting the wound size.

9. Method for providing a cut-to-size and liquid-permeable wound dressing (3p) for vacuum therapy of wounds, the method comprising the steps of
a) providing a cutting-to-size aid (10) according to one of Claims 1 to 8,
b) providing a liquid-permeable wound dressing (3),
c) tracing an outline of the wound on the cutting-to-size aid (10),
d) adhesively fixing the second side of the cutting-to-size aid onto that side of the liquid-permeable wound dressing (3) facing away from the wound during use, such that a flat combination of wound dressing (3) and cutting-to-size aid (10) is obtained,
e) cutting to size the flat combination of wound dressing (3) and cutting-to-size aid (10), wherein the cutting to size is performed along the outline drawn onto the cutting-to-size aid (10) in step c).

10. Method for providing a cut-to-size and liquid-permeable wound dressing (3p) for vacuum therapy of wounds according to Claim 9, further comprising the step f) of removing the cutting-to-size aid (10) from the cut-to-size wound dressing (3p).

11. Method for providing a cut-to-size and liquid-permeable wound dressing (3p) for vacuum therapy of wounds according to Claim 9 or 10, wherein the wound outline is traced onto the cutting-to-size aid (10) while the cutting-to-size aid (10) is held on or placed onto the wound.

12. Device according to Claims 1 to 8 or cut-to-size and liquid-permeable wound dressing (3p) provided according to the methods of Claims 9 to 11 for use in the vacuum therapy of wounds.

13. Ready-to-use set for vacuum therapy of wounds, comprising
a) an air-impermeable cover material (2) for air-tight closure of the wound and of the wound environment,
b) a means (4, 5) for functionally connecting the wound space to a vacuum source (7) located outside the cover material (2), such that a vacuum can be produced in the wound space and liquids can be suctioned from the wound space,
c) a liquid-permeable wound dressing (3) for insertion into the gap formed between wound surface and cover material (2),
d) a cutting-to-size aid (10) comprising a transparent film (8) with a first and a second side, which transparent film (8) is inscribable on its first side and has, on its second side, a self-adhesive coating (9), wherein the components a) to d) are packaged in a sterile state.

## Revendications

1. Dispositif pour la thérapie par dépressurisation de plaies, comprenant
a) un matériau de recouvrement imperméable à l'air (2) pour la fermeture à l'abri de l'air de la plaie et du voisinage de la plaie (1),
b) un moyen (4, 5) pour relier de façon fonctionnelle la zone de la plaie à une source de dépression se trouvant à l'extérieur du matériau de recouvrement, de telle manière qu'une dépression puisse être établie dans la zone de la plaie et que des liquides puissent être aspirés hors de la zone de la plaie,
c) un pansement perméable au liquide (3), à appliquer dans l'espace intermédiaire qui est formé entre la surface de la plaie (W) et le matériau de recouvrement (2),
**caractérisé en ce que** le dispositif comprend en outre
d) une aide à la découpe (10) pour le pansement perméable au liquide (3), dans lequel
d1) l'aide à la découpe (10) comprend une feuille transparente (8) avec un premier côté et un deuxième côté,
d2) la feuille transparente (8) peut recevoir des inscriptions sur son premier côté, de telle manière que le contour d'une plaie puisse être tracé sur la feuille transparente (8),
d3) la feuille transparente (8) présente un revêtement autoadhésif (9) au moins sur le deuxième côté situé à l'opposé du premier côté, de telle manière que l'aide à la découpe (10) puisse être appliquée par adhérence sur le côté du pansement (3) situé, en utilisation, à l'opposé de la plaie.

2. Dispositif pour la thérapie par dépressurisation de plaies selon la revendication 1, dans lequel la feuille transparente (8) est revêtue d'un adhésif polyacrylate sur le deuxième côté.

3. Dispositif pour la thérapie par dépressurisation de plaies selon la revendication 1 ou 2, dans lequel la feuille transparente (8) est une feuille de polymère.

4. Dispositif pour la thérapie par dépressurisation de plaies selon la revendication 3, dans lequel le polymère de la feuille de polymère comprend le polyuréthane, le polyester, le polypropylène, le polyéthylène, le polyamide, le chlorure de polyvinyle, le polyorganosiloxane ou un mélange de ceux-ci.

5. Dispositif pour la thérapie par dépressurisation de plaies selon l'une quelconque des revendications précédentes, dans lequel l'aide à la découpe (10) comprend en outre au moins une feuille de recouvrement transparente (11, 12) sur le côté tourné, en utilisation, vers la plaie.

6. Dispositif pour la thérapie par dépressurisation de plaies selon la revendication 5, dans lequel ladite au moins une feuille de recouvrement (11, 12) comprend un film de polyester siliconé.

7. Dispositif pour la thérapie par dépressurisation de plaies selon l'une quelconque des revendications précédentes, dans lequel l'aide à la découpe (10) comprend au moins une autre feuille sur le côté tourné, en utilisation, à l'opposé de la plaie.

8. Dispositif pour la thérapie par dépressurisation de plaies selon la revendication 7, dans lequel ladite au moins une autre feuille comprend un film de polyester transparent (15) avec un premier côté et un deuxième côté, et dans lequel en outre
d2) le film de polyester transparent (15) peut recevoir des inscriptions sur son premier côté, de telle manière que le contour d'une plaie puisse être tracé sur le film de polyester transparent (15), d3) le film de polyester transparent (15) présente un revêtement autoadhésif (16) sur le deuxième côté situé à l'opposé du premier côté, de telle manière que le film de polyester transparent (15) puisse être appliqué par adhérence sur le premier côté de la feuille transparente (8) et que le film de polyester transparent (15) puisse en outre être retiré de la feuille transparente (8) après la découpe du pansement (3) et puisse être archivé pour la documentation de la taille de la plaie à titre d'aide à la documentation.

9. Procédé de préparation d'un pansement découpé (3p) perméable au liquide pour la thérapie par dépressurisation de plaies, comprenant les étapes de:
a) préparer une aide à la découpe (10) selon l'une quelconque des revendications 1 à 8,
b) préparer un pansement perméable au liquide (3),
c) tracer un contour de plaie sur l'aide à la découpe (10),
d) coller le deuxième côté de l'aide à la découpe sur le côté du pansement perméable au liquide (3) situé, en utilisation, à l'opposé de la plaie, de telle manière qu'il se forme un assemblage plat du pansement (3) et de l'aide à la découpe (10),
e) découper l'assemblage plat du pansement (3) et de l'aide à la découpe (10), dans lequel la découpe est effectuée le long du contour tracé à l'étape c) sur l'aide à la découpe (10).

10. Procédé de préparation d'un pansement découpé (3p) perméable au liquide pour la thérapie par dépressurisation de plaies selon la revendication 9, comprenant en outre l'étape f) d'enlèvement de l'aide à la découpe (10) du pansement découpé (3p).

11. Procédé de préparation d'un pansement découpé (3p) perméable au liquide pour la thérapie par dépressurisation de plaies selon la revendication 9 ou 10, dans lequel le tracé du contour de la plaie sur l'aide à la découpe (10) est effectué pendant que l'aide à la découpe (10) est maintenue sur la plaie ou est posée sur la plaie.

12. Dispositif selon la revendication 1 à 8 ou pansement découpé (3p) perméable au liquide préparé par le procédé des revendications 9 à 11 pour l'utilisation dans la thérapie par dépressurisation de plaies.

13. Ensemble prêt à l'emploi pour le traitement de plaies par dépressurisation comprenant:
a) un matériau de recouvrement imperméable à l'air (2) pour la fermeture à l'abri de l'air de la plaie et du voisinage de la plaie,
b) un moyen (4, 5) pour relier de façon fonctionnelle la zone de la plaie à une source de dépression (7) se trouvant à l'extérieur du matériau de recouvrement (2), de telle manière qu'une dépression puisse être établie dans la zone de la plaie et que des liquides puissent être aspirés hors de la zone de la plaie,
c) un pansement perméable au liquide (3), à appliquer dans l'espace intermédiaire qui est formé entre la surface de la plaie et le matériau de recouvrement (2),
d) une aide à la découpe (10) comprenant une feuille transparente (8) avec un premier côté et un deuxième côté, qui peut recevoir des inscriptions sur son premier côté et qui présente un revêtement autoadhésif (9) sur son deuxième côté,
dans lequel les composants a) à d) se présentent sous un emballage stérile.
